# EUROPÄISCHE PATENTSCHRIFT

(11) **EP 0 882 032 B1**
(45) Veröffentlichungstag und Bekanntmachung des Hinweises auf die Patenterteilung: **04.06.2003**
(21) Anmeldenummer: 96943900.9
(22) Anmeldetag: 07.12.1996
(51) Int. Cl.: C07D 307/33

(54) **VERFAHREN ZUR HERSTELLUNG VON GAMMA-BUTYROLACTON SOWIE DESSEN VERWENDUNG**
PROCESS FOR THE PREPARATION OF GAMMA-BUTYROLACTONE AND THE USE THEREOF
PROCEDE DE PREPARATION DE GAMMA-BUTYROLACTONE AINSI QUE SON UTILISATION

(30) Priorität: 27.12.1995 DE 19548818
(43) Veröffentlichungstag der Anmeldung: 09.12.1998
(73) Patentinhaber: Akzo Nobel N.V., 6824 BM Arnhem (NL)
(72) Erfinder: BERGFELD, Manfred, Josef, D-63906 Erlenbach (DE); UIHLEIN, Kurt, D-63920 Gro heubach (DE)
(74) Vertreter: Fett, Günter
(86) Internationale Anmeldenummer: EP9605486
(87) Internationale Veröffentlichungsnummer: WO97024346

(56) Entgegenhaltungen:
- EP-A- 0 638 565
- WO-A-91/16132
- DE-A- 2 404 493
- US-A- 3 065 243
- US-A- 4 006 165
- CHEMICAL ABSTRACTS, vol. 82, no. 21, 26.Mai 1975 Columbus, Ohio, US; abstract no. 139947a, Seite 611; XP002028723 & JP 49 020 585 A (MITSUBISHI CHEMICAL IND. CO., LTD.) 25.Mai 1974 in der Anmeldung erwähnt

## Beschreibung

Die Erfindung betrifft ein Verfahren zur Herstellung von gamma-Butyrolacton durch katalytische Hydrierung von Maleinsäureanhydrid, Bernsteinsäureanhydrid oder deren Säuren in der Dampfphase sowie dessen Verwendung zur Herstellung von Pyrrolidonen und N-Alkylpyrrolidonen.

Gamma-Butyrolacton ist eine wichtige Chemikalie, die als Ausgangsstoff für zahlreiche Synthesen von Bedeutung ist. Sie spielt zum Beispiel eine Rolle bei der Herstellung von Buttersäure und deren Derivaten, 1,4-Butandiol, Tetrahydrofuran, N-Methylpyrrolidon, Polyvinylpyrrolidon, Methionin und dergleichen. Gamma-Butyrolacton ist ferner ein wichtiges Lösungsmittel unter anderem für Acrylate und Polymere auf Styrolbasis. Es kann ferner als Lösungsmittel unter anderem bei der Herstellung von Synthese-Fasern eingesetzt werden.

Eine Reihe von Herstellungsmethoden gehen von Maleinsäureanhydrid bzw. Abkömmlingen wie Maleinsäure, Bernsteinsäureanhydrid, Maleinsäureester aus, die einer Hydrierung unterworfen werden. Die Hydrierung wird meistens in der Dampfphase und in Gegenwart von Katalysatoren durchgeführt. Es werden in der Patentliteratur zahlreiche Katalysatoren für diese Reaktionen beschrieben. So ist zum Beispiel der US-PS 3 065 243 ein Verfahren zu entnehmen, bei dem Kupferchromit als Katalysator dient. Wie der Beschreibung und den Beispielen dieser Patentschrift zu entnehmen ist, entstehen bei dieser Umsetzung noch beträchtliche Mengen an Bernsteinsäureanhydrid, das im Kreislauf gefahren werden muß.

Es hat nicht an Versuchen gefehlt, Katalysatoren zu entwickeln, um die Ausbeute und die Selektivität zu verbessern. Auch war es Ziel der Untersuchungen, die Lebensdauer der Katalysatoren zu verbessern, da bei vielen Katalysatoren die Standzeiten für den Dauerbetrieb zu kurz sind; es kommt bei Dauerbetrieb nämlich zu schnell zu einer Desaktivierung des Katalysators.

So werden in der kanadischen Patentschrift Nr. 840 452 weiterentwickelte Katalysatoren beschrieben, die auf der Basis von Kupfer/Zink aufgebaut sind. Diese können zusammen mit Asbest zu entsprechenden Katalysatorteilchen verarbeitet werden. Sowohl der in dieser kanadischen Patentschrift beanspruchte Katalysator als auch der vergleichsweise hergestellte Kupferchromitasbest-Katalysator erfüllen noch nicht alle Wünsche, die an einen guten Kätalysator für die Herstellung von gamma-Butyrolacton gestellt werden.

In der DE-OS 24 04 493 wird ein Verfahren beschrieben, bei dem in Gegenwart von Wasserdampf hydriert wird. Dadurch soll die Verkokung des Katalysators reduziert werden. Von Nachteil bei diesem Verfahren ist unter anderem, daß zusätzlich Wasser eingeführt wird, das sowieso schon als Nebenprodukt gebildet wird, was dieses Verfahren aufwendiger macht.

Weitere Katalysatoren auf Basis von Kupferchromit werden zum Beispiel in der US-PS 4 006 165 beschrieben, wobei dieser Katalysator noch Nickel enthalten muß. Diese Katalysatoren können auf Aluminiumoxyd oder Silica wie Kieselgur aufgebracht werden oder auch durch Mischung mit denselben hergestellt werden.

In der EP-A1-0 638 565 wird ein Verfahren beschrieben, bei dem gamma-Butyrolacton durch katalytische Hydrierung von Maleinsäureanhydrid in der Dampfphase in Gegenwart von Katalysatoren auf der Basis von Kupferchromit in reduzierter Form hergestellt wird. Obwohl mit dem dort eingesetzten einheitlichen, d.h. homogenen Katalysator auf der Basis der drei Komponenten Kupferoxid, Chromoxid und Siliziumdioxid hohe Selektivitäten und gute Ausbeuten erreicht werden, hat es sich gezeigt, daß die Dauerbelastbarkeit des Katalysators noch zu wünschen übrig läßt.

In der US-PS 5 347 021 wird ein Verfahren zur Herstellung von gamma-Butyrolacton beschrieben, bei dem ebenfalls in der Dampfphase Wasserstoff und Maleinsäureanhydrid in Gegenwart eines Katalysators umgesetzt werden. Der Katalysator ist auf der Basis der Bestandteile Kupferoxid, Zinkoxid, Aluminiumoxid und Graphit aufgebaut. Obwohl das Verfahren mit verhältnismäßig guten Selektivitäten und Ausbeuten arbeitet, muß bei dem dort beschriebenen Verfahren nach etwa 100 Std. Laufzeit der Katalysator reaktiviert werden.

Wenn auch bereits zahlreiche Katalysatoren für die Umsetzung von Wasserstoff und Maleinsäureanhydrid etc. zu gamma-Butyrolacton beschrieben sind, besteht noch ein Bedürfnis nach Katalysatoren, mit denen sich diese Umsetzung auf noch bessere und vorteilhaftere Weise durchführen läßt.

Aufgabe der Erfindung ist es deshalb, ein Verfahren zur Herstellung von gamma-Butyrolacton durch Hydrierung von Maleinsäureanhydrid, Bernsteinsäureanhydrid oder deren Säuren in der Dampfphase in Gegenwart eines Katalysators zur Verfügung zu stellen, das mit hohen Ausbeuten arbeitet, das eine hervorragende Selektivität besitzt, das sowohl unter erhöhtem Druck als auch bei normalem oder unteratmosphärischem Druck arbeiten kann, das wirtschaftlich arbeitet, das flexibel ist und das insbesondere Vorteile bei der Aufarbeitung des Reaktionsprodukts und bei der Rückführung bestimmter Komponenten in den Kreislauf bietet und das auch so gefahren werden kann, daß ein Rezyklieren von Bernsteinsäureanhydrid, das ggf. als Zwischenstufe entsteht, nicht erforderlich wird.

Aufgabe der Erfindung ist es ferner ein Verfahren zur Verfügung zu stellen, das über längere Zeit betrieben werden kann, ohne daß es zu einer Desaktivierung des Katalysators kommt.

Diese Aufgabe wird gelöst durch ein Verfahren zur Herstellung von gamma-Butyrolacton durch katalytische Hydrierung von Maleinsäureanhydrid, Bernsteinsäureanhydrid oder deren Säuren in der Dampfphase in Gegenwart von Katalysatoren auf der Basis von Kupferoxid und Aluminiumoxid in reduzierter Form, dadurch gekennzeichnet, daß man zur Durchführung der Reaktion einen Katalysator verwendet, der auf der Basis von 50 bis 95 Gew% Kupferoxid, 3 bis 30 Gew% Aluminiumoxid und 0 bis 25 Gew% eines Binders aufgebaut ist. Vorzugsweise ist der verwendete Katalysator auf Basis von 80 bis 90 % Kupferoxid, 5 bis 15 % Aluminiumoxid und in dem zu 100 % fehlenden Rest aus einem Binder aufgebaut. Als Binder sind insbesondere Graphit und Kieselgel geeignet. Ein besonders vorteilhafter Katalysator für das Verfahren ist auf der Basis von 83,5 - 85,5 Gew% Kupferoxid, 9 - 11 Gew% Aluminiumoxid und 4,5 - 6,5 Gew% Graphit aufgebaut. Es ist vorteilhaft, wenn der Katalysator eine einheitliche Struktur aufweist, d.h. homogen aufgebaut ist. Der Katalysator kann auch auf einem Träger aufgebracht sein.

Man kann die Hydrierung auch in Gegenwart eines inerten Gases als Verdünnungsmittel, vorzugsweise Stickstoff, durchführen. Außer Stickstoff können auch die bekannten Edelgase wie Argon, Krypton, Helium oder deren Gemische unter sich oder mit Stickstoff verwendet werden.

Es versteht sich von selbst, daß der Katalysator auf Basis der 3 Komponenten in an sich bekannter Weise vor dem Einsatz in der Reaktion in üblicher Weise reduziert wird. Vorzugsweise wird die Reduktion im Reaktor selbst durchgeführt.

Die Reduktion kann zum Beispiel nach folgender Verfahrensweise durchgeführt werden. Der Katalysator, der als Katalysator-Bett vorliegt, wird im Reaktor im Stickstoffstrom auf 150 °C aufgeheizt. Bei dieser Temperatur wird langsam Wasserstoff bis zu einer Eingangskonzentration von bis zu 8 Vol% eingespeist. Die Temperatur sollte dabei im Katalysator-Bett nicht stärker als um 25°C steigen.

Nach Abklingen der Reaktionswärme wird die Wasserstoff-Konzentration auf 80 - 100 % durch Zurücknahme des Stickstoffstromes gesteigert und die Temperatur bis zu 280°C angehoben. Die Temperatur wird 12 Stunden unter H₂-Durchfluß eingehalten; diesen Vorgang nennt man im allgemeinen Nachreaktion.

Selbstverständlich kann die Reduktion auch auf andere Weise geschehen, zum Beispiel wie in der US-PS 3 065 243 in Spalte 2, Zeilen 54 bis 66 angegeben.

Einheitlicher Katalysator im Rahmen der Erfindung bedeutet, daß die Komponenten so innig miteinander verbunden sind, daß der Katalysator eine einheitliche Struktur aufweist, d.h. im wesentlichen homogen ist und keine größeren heterogenen unterschiedlich aufgebauten Bestandteile aufweist.

Der Katalysator kann dann sogleich in den Reaktor eingebracht und nach entsprechender Reduktion für die Reaktion verwendet werden.

Die Hydrierung von Maleinsäureanhydrid, d.h. die Reaktion von Maleinsäureanhydrid mit Wasserstoff wird in der Dampfphase durchgeführt, d.h. bei erhöhten Temperaturen zum Beispiel in einem Bereich von etwa 100 - 400 °C, bevorzugt ist der Bereich von etwa 260 - 320 °C.

Das dampfförmige Maleinsäureanhydrid kann für sich selbst durch Erhitzen und Überführen in die Dampfphase und entsprechendes Dosieren in den Reaktionsraum geführt werden. Es ist aber auch möglich, die erforderlichen Mengen an Maleinsäureanhydrid-Dämpfen mittels des zu dosierenden Wasserstoffstromes in den Reaktor mitzuführen. Selbstverständlich kann dies auch durch das gegebenenfalls mitverwendete inerte Gas wie Stickstoff geschehen.

Das molare Verhältnis von Maleinsäureanhydrid zu Wasserstoff kann in dem Strom der Ausgangsprodukte in weiten Bereichen variieren und zum Beispiel zwischen 1 : 20 bis 1 : 250 liegen. Der Bereich 1 : 40 bis 1 : 100 wird bevorzugt.

Die Reaktion kann sowohl bei normalem Druck als auch bei Unterdruck oder erhöhtem Druck durchgeführt werden wie zum Beispiel zwischen 0,1 bis 10 bar.

Inertes Gas bedeutet im Rahmen der Erfindung, daß es bei der Umsetzung nicht als Reaktionspartner oder Reaktionsprodukt teilnimmt und sich auch nicht selbst durch eine Reaktion verändert.

Durch Verwendung eines inerten Gases als Verdünnungsmittel ist es möglich, die Reaktion in günstiger Weise zu beeinflussen. Das Verhältnis von inertem Gas, zu dem vorzugsweise Stickstoff zu zählen ist, das aber auch ein Edelgas oder Kohlendioxid sein kann, bzw. ein Gemisch dieser Gase zu Wasserstoff kann ebenfalls in weiten Bereichen variieren. Es versteht sich von selbst, daß die Verdünnung, abhängig von den gewählten sonstigen Reaktionsbedingungen dort ihre Grenze findet, wo der Anteil des Verdünnungsmittels so groß ist, daß zu wenig Wasserstoff vorhanden ist und die Ausbeute bezogen auf Maleinsäueanhydrid stark abnimmt. Diese Grenze kann durch wenige einfache Versuche, die zum handwerklichen Können eines Durchschnittsfachmannes gehören, ermittelt werden.

Es ist möglich, die Reaktion durch Variieren der verschiedensten Verfahrensparameter zu beeinflussen. So kann die Verweilzeit geändert werden, was durch Einstellen verschiedener Dosiergeschwindigkeiten ermöglicht werden kann, was aber auch durch Verlängerung der Reaktionsstrecke bewerkstelligt werden kann, zum Beispiel durch Verwendung eines längeren Reaktionsrohres, das entsprechend mit Katalysator gefüllt ist. Zur beschleunigten Abführung der Reaktionswärme kann der Katalysator ganz oder teilweise mit einem im Reaktionsmedium inerten Material mit ausreichend hoher Wärmeleitfähigkeit verdünnt werden, wozu sich z.B. Stahlkugeln, Steatit usw. eignet.

Die Reaktion läßt sich so steuern, daß das als Zwischenstufe entstehende Bernsteinsäureanhydrid am Ende des Reaktors nicht mehr vorhanden ist und somit nicht mehr im Kreislauf gefahren werden muß. Andererseits läßt sich bei Bedarf die Reaktion auch so steuern, daß Bernsteinsäureanhydrid in mehr oder weniger großen Mengen noch in den austretenden Reaktionsprodukten vorhanden ist und dann entweder nach Abtrennung für sich selbst weiterverarbeitet wird oder auch wieder im Kreislauf gefahren wird.

Das gamma-Butyrolacton und das gebildete Wasser werden in an sich bekannter Weise getrennt.

Es war völlig überraschend, daß die erfindungsgemäße Verwendung des Katalysators sowohl eine hohe Selektivität als auch eine hohe Ausbeute bewirkt. Die Vorteile des erfindungsgemäß verwendeten Katalysators zeigen sich nicht nur beim Arbeiten mit oder ohne Verdünnungsmittel, sie zeigen sich auch, wenn man die molaren Verhältnisse der Reaktionspartner verändert, und wenn man die Temperaturen variiert. Somit ist dieser Katalysator bei der Herstellung von gamma-Butyrolacton durch Reduktion von Maleinsäureanhydrid mittels Wasserstoff unter den verschiedensten Verfahrensbedingungen mit großen Vorteilen zu verwenden.

Es war ferner völlig überraschend, daß die erfindungsgemäße Verwendung des Katalysators ein Verfahren mit sehr langen Katalysatorstandzeiten erlaubt, ohne daß es zu einer Desaktivierung kommt. Es ist also nicht erforderlich, den Katalysator nach verhältnismäßig kurzen Laufzeiten zu erneuern oder wieder zu aktivieren, was zu Unterbrechungen führen würde und einen erheblichen Aufwand bedeuten würde.

Von besonderem Vorteil ist, daß das bei dem Verfahren gemäß der Erfindung entstehende gamma-Butyrolacton direkt ohne Abtrennung des gebildeten Wassers zur Herstellung von Pyrrolidon und N-Alkylpyrrolidonen in der Flüssigphase unter hohem Druck, insesondere zur Herstellung von N-Methylpyrrolidon durch Zusatz von Methylamin verwendet werden kann.

Hierbei kann das klassische Aminierungsverfahren wie z.B. in Ullmann's, Encyclopedia of Industrial Chemistry, 5. überarbeitete Ausgabe, Band A22, Seite 457 - 459 oder JP 49-0585 beschrieben, angewandt werden.

Die Erfindung wird an Hand folgender Beispiele näher erläutert:

### Beispiel 1 - (Atmosphärischer Druck ohne Wasserstoffrecyclen)

Ein Katalysator mit der Zusammensetzung 77 % Kupferoxid (CuO), 9 % Aluminiumoxid (Al₂O₃), 5 % Graphit und 9 % flüchtige Bestandteile, insbesondere Hydratwasser, käuflich erhältlich bei der Firma Mallinckrodt Chemical GmbH, D 53761 Hennef/Sieg wird zunächst von seinen flüchtigen Bestandteilen befreit und reduziert. Die Reduktion des Katalysators erfolgte in folgender Weise:

Der Katalysator wird im Stickstoffstrom auf 150°C aufgeheizt. Bei dieser Temperatur wird langsam Wasserstoff bis zu einer Eingangskonzentration von bis zu 8 Volumenprozent eingespeist. Der Temperaturanstieg der Schüttung wird dabei auf unter 25°C gehalten. Nach Abklingen der Reaktionswärme wird der Wasserstoffgehalt von 80 % auf 100% gesteigert und die Temperatur auf bis zu 280°C angehoben. Die Temperatur wird 2 Stunden unter Wasserstoffluß beibehalten (Nachreaktion).

### Durchführung der Umsetzung

Hierauf wird Maleinsäureanhydrid und Wasserstoff in gewünschter Menge gasförmig zudosiert, d.h. 1 Mol pro Stunde Wasserstoff und 0,01 Mol pro Stunde Maleinsäureanhydrid. Die Temperatur der Beheizung wird auf 265°C eingestellt. Dabei wird ein Hot-Spot von 7°C beobachtet. Die Ausbeute betrug bei 100%igem Umsatz 98%.

### Beispiel 2 - (Kontinuierliches Verfahren unter Druck mit Wasserstoffrecyclen)

In einem Edelstahlrohrreaktor mit einem Innendurchmesser von 30 mm und einer Länge von 1,2 m werden 660 g Katalysator, wie in Beispiel 1 beschrieben, unzerkleinert eingesetzt. Von den eben genannten 660 g Katalysator werden 160 g mit 160 g eines aus Stahlkugeln bestehenden Inertmaterials gemischt, um die Temperatur im Bereich des Hot Spots zu reduzieren. Diese Mischung bildet den oberen Teil der Katalysatorschüttung. Die Wärmeabfuhr erfolgt über ein Wärmeträgeröl, das den Doppelmantel des Reaktorrohres durchströmt. Der zur Hydrierung eingesetzte Wasserstoff wird recycelt, nachdem die Produkte kondensiert wurden (Kondensation bei 25°C). Die kondensierten Produkte werden direkt einer Analyse zugeführt. Ca. 10 % der für die eigentliche Umsetzung benötigten Wasserstoffmenge wird ausgeschleußt, um eine Anreicherung von Nebenprodukten im Kreisgas zu verringern. Der Druck beträgt 6 bar absolut.

Bei dem Verfahren wird nach der Reduktion ein Wasserstoffstrom durch den Reaktor von 1000 nl/h eingestellt, wobei nl Normliter bedeutet. Es werden 60 g/Stunde Maleinsäureanhydrid zudosiert. Die frisch zudosierte Wasserstoffmenge beträgt 60 nl/h. Die ausgeschleußte Gasmenge wird über einen Druckregler gesteuert. Die Zudosierung des Maleinsäureanhydrids erfolgt flüssig in einem dem eigentlichen Reaktor vorgeschalteten Verdampfer. Die ausgeschleußte Gasmenge wird über einen Druckregler gesteuert. Der Druck beträgt 6 bar.

Die Ausbeute blieb bei einer Temperatur des Wärmeträgermediums von 277°C und einer max. im Reaktor gemessenen Temperatur von 304°C über einen Zeitraum von 1600 Stunden konstant bei ca. 92 % (siehe Tabelle 1).

**Tabelle 1**

| Zeit (h) | Ausbeute (%) |
|---|---|
| 0 | 92,1 |
| 200 | 92,2 |
| 400 | 92,8 |
| 600 | 92,6 |
| 800 | 92,9 |
| 1100 | 93,3 |
| 1600 | 92,8 |

### Beispiel 3 - (Vergleichsbeispiel)

Zum Nachweis der völlig überraschenden, überlegenen Reaktionsweise des erfindungsgemäßen Katalysators werden sieben verschiedene Katalysatoren auf Basis Kupferchromit vorgestellt, die übliche bekannte Kupferchromit-Katalysatoren darstellen, während Katalysator 8 dem erfindungsgemäßen Katalysator entspricht. Die Katalysatoren werden durch Fällen entsprechender Lösungen gewonnen. Die Zusammensetzung der eingesetzten Katalysatoren ist in Tabelle 2 gegeben, wobei die Zusammensetzung diejenige vor der Reduktion ist.

**Tabelle 2**

| Zusammensetzung (vor Reduktion) | Kat.-Nr. |
|---|---|
| 2 CuO • Cr₂O₃* | 1 |
| Cu-chromit (9.7 % BaO) | 2 |
| 80 % CuO, 20 % Cr₂O₃ | 3 |
| 78 % CuO, 20 % Cr₂O₃, 2% SiO₂ | 4 |
| 42 % CuO, 38 % Cr₂O₃ | 5 |
| 33 % CuO, 38 % Cr₂O₃, 9 BaO | 6 |
| 76 % CuO, 24 % Cr₂O₃ | 7 |
| 84,6 % CuO, 9,9 % Al₂O₃ 5,5 % Graphit** | 8 |

| | |
|---|---|
| * Handelsprodukt E-113 T der Firma Mallinckrodt | |
| ** Handelsprodukt E-408 der Firma Mallinckrodt | |

Die Ausführung der Reduktion erfolgte nach folgender an sich üblicher Verfahrensweise:

Das Katalysatorbett, das sich bereits in dem vorgesehenen Reaktor befindet, wird im Stickstoffstrom auf 150°C aufgeheizt. Bei dieser Temperatur wird langsam Wasserstoff bis zu einer Eingangskonzentration von bis zu 8 Vol% eingespeist. Der Temperaturanstieg der Schüttung sollte nicht größer als 25°C sein (Reduktionsschritt).

Nach Abklingen der Reaktionswärme wird die Wasserstoffkonzentration auf 80 bis 100% gesteigert und die Temperatur auf bis zu 280°C angehoben. Die Temperatur wird 12 h unter H₂-Durchfluß eingehalten (Nachreduktion).

### Verfahrensdurchführungen:

Die Katalysatorpellets werden zerkleinert und eine Fraktion von 0,8 bis 1,2 mm ausgewählt. Diese werden in ein mit Silikonöl beheizbares Quarzglasrohr mit 1 cm Innendurchmesser und 30 cm Länge gegeben. Nach Durchführung des oben beschriebenen Reduktionsschrittes erfolgt die Versuchsausführung. Wasserstoff wird über einen Massendurchflußregler dosiert und der Maleinsäureanhydridpartialdruck über einen sogenannten Sättiger eingestellt. Dies erfolgt dadurch, daß Wasserstoff und ggf. Stickstoff durch den Sättiger, in dem sich flüssiges Maleinsäureanhydrid befindet, geleitet werden, wobei sich aufgrund der genau fixierten Temperatur ein bekannter Maleinsäureanhydrid-Partialdruck einstellt. Das Gemisch wird über beheizte Leitungen dem Reaktor zugeführt.

Die Katalysatoren wurden reduziert wie oben beschrieben. Jeweils 20 ml Schüttvolumen des reduzierten Katalysators wurden in den Reaktor gefüllt und bei einem Durchfluß von 0,38 mol N₂/h und 2 mol H₂/h auf eine Reaktionstemperatur von 275°C gebracht. Dann wurde das Gasgemisch durch den Sättiger geleitet und so ein Molenstrom an Maleinsäureanhydrid von 0,02 mol/h eingestellt. Nach 2 h Reaktionszeit wurde das Produktgemisch analysiert. Die Ergebnisse sind in der folgenden Tabelle 3 wiedergegeben.

**Tabelle 3**

| Kat- Nr. | Ausbeuten (in %) | Ausbeuten (in %) | Umsatz (in %) |
|---|---|---|---|
| | gamma-Butyrolacton | Bernsteinsäure-anhydrid | Maleinsäure anhydrid |
| 1 | 22.1 | 77,6 | 100 |
| 2 | 49.8 | 49,6 | 100 |
| 3 | 64.9 | 34,2 | 100 |
| 4 | 98.1 | - | 100 |
| 5 | 25.0 | 62 | 100 |
| 6 | 4.7 | 14,0 | 19,5 |
| 7 | 90.9 | 6,9 | 100 |
| 8 | 98,2 | 0 | 100 |

Daraus ist die besondere Qualität der Katalysatoren Nr. 4 und 8 zu erkennen, wobei von besonderer Bedeutung ist, daß auch das Zwischenprodukt Bernsteinsäureanhydrid vollständig umgesetzt wird, ansonsten sind häufig technische Probleme mit der Kristallisation des Bernsteinsäureanhydrids zu erwarten, die sich in Verstopfung von Rohrleitungen usw. auswirkt. Der Nachteil von Katalysator Nr. 4 gegenüber dem erfindungsgemäßen Katalysator liegt in seiner kürzeren Dauerstandfestigkeit, wie das folgende Beispiel zeigt.

### Beispiel 4 - (Vergleichsbeispiel)

Der unerwartete Vorteil des erfindungsgemäßen Katalysators gegenüber Katalysator 4 liegt in seiner wesentlich höheren Langzeitstabilität. Bereits innerhalb weniger Stunden desaktivierte Katalysator Nr. 4 (s. Tabelle 4), während der erfindungsgemäße Katalysator über 1600 Stunden stabil blieb (S. Tabellen 1 und 4). Die Vorgehensweise, Katalysatormasse, Reaktionsbedingungen und Verdünnung sind identisch mit Beispiel 2. Zum besseren Vergleich wurden die Ergebnisse, die mit dem erfindungsgemäßen Katalysator erzielt wurden, in Tabelle 4 noch einmal mit aufgenommen.

**Tabelle 4**

| | Ausbeute [%] | |
|---|---|---|
| Zeit [h] | Katalysator Nr. 8 | Katalysator Nr. 4 |
| 0 | 92,1 | 91,1 |
| 20 | 92,5 | 89,2 |
| 40 | 92,7 | 85,4 |
| 100 | 92,4 | 82,6 |
| 200 | 92,2 | 73,1 |
| 400 | 92,8 | 56,4 |

### Beispiel 5 - (Herstellung von N-Methylpyrrolidon)

12,8 g des Reaktionsproduktgemisches aus Beispiel 2 (0,11 mol gamma-Butyrolacton) werden mit 14 g 40 % wässriger Monomethylaminlösung (0,19 mol Monomethylamin) gemischt und in einen Hochdruckautoklaven gegeben. Das Gemisch wird auf Reaktionstemperatur (290°C) gebracht, wobei der Druck auf 77 bar steigt. Nach einer Reaktionsdauer von zwei Stunden sind mehr als 99 % des gamma-Butyrolacton umgesetzt. Die Ausbeute an N-Methylpyrrolidon beträgt 99,1 % bezogen auf gamma-Butyrolacton und 91,8 % bezogen auf Maleinsäureanhydrid. Die Durchführung des Experiments entspricht der klassischen Herstellungsmethode von NMP wie zum Beispiel in JP 49-20585 beschrieben, es sind jedoch noch alle Reaktionsprodukte aus der 1. Reaktionsstufe wie z.B. Wasser oder Butanol im Eduktgemisch enthalten.

## Patentansprüche

1. Verfahren zur Herstellung von gamma-Butyrolacton durch katalytische Hydrierung von Maleinsäureanhydrid, Bernsteinsäureanhydrid oder deren Säuren in der Dampfphase in Gegenwart von Katalysatoren auf der Basis von Kupferoxid und Aluminiumoxid in reduzierter Form, **dadurch gekennzeichnet, daß** man zur Durchführung der Reaktion einen Katalysator verwendet, der auf der Basis von 50 bis 95 Gew% Kupferoxid, 3 bis 30 Gew% Aluminiumoxid und 0 bis 25 Gew% eines Binders aufgebaut ist.

2. Verfahren nach Anspruch 1, **dadurch gekennzeichnet, daß** der Katalysator auf Basis von 80 bis 90 % Kupferoxid, 5 bis 15 Gew% Aluminiumoxid und in dem zu 100 Gew% fehlenden Rest aus einem Binder aufgebaut ist.

3. Verfahren nach einem der Ansprüche 1 bis 2, **dadurch gekennzeichnet, daß** man als Binder Graphit verwendet.

4. Verfahren nach Anspruch 2 oder 3, **dadurch gekennzeichnet, daß** der Katalysator auf der Basis von 83,5 bis 85,5 Gew% Kupferoxid, 9 - 11 Gew% Aluminiumoxid und 4,5 bis 6,5 Gew% Graphit aufgebaut ist.

5. Verfahren nach einem der Ansprüche 1 bis 2, **dadurch gekennzeichnet, daß** man als Binder Kieselgel verwendet.

6. Verfahren nach einem der Ansprüche 1 bis 5, **dadurch gekennzeichnet, daß** der Katalysator eine einheitliche Struktur aufweist.

7. Verfahren nach einem der Ansprüche 1 bis 6, **dadurch gekennzeichnet, daß** der Katalysator auf einem Träger aufgebracht ist.

8. Verfahren nach einem der Ansprüche 1 bis 7, **dadurch gekennzeichnet, daß** man die Hydrierung in Gegenwart von reinem Wasserstoff durchführt.

9. Verfahren nach einem der Ansprüche 1 bis 8, **dadurch gekennzeichnet, daß** man die Hydrierung in Gegenwart eines inertes Gases als Verdünnungsmittel durchführt.

10. Verfahren nach Anspruch 9, **dadurch gekennzeichnet, daß** man als Verdünnungsmittel Stickstoff verwendet.

11. Verfahren nach einem der Ansprüche 1 bis 10, **dadurch gekennzeichnet, daß** der Druck zwischen 0,1 und 10 bar beträgt.

12. Verfahren nach Anspruch 11, **dadurch gekennzeichnet, daß** der Druck 4 - 7 bar beträgt.

13. Verfahren nach einem der Ansprüche 1 bis 12, **dadurch gekennzeichnet, daß** das molare Wasserstoff : Maleinsäureanhydrid-, Bernsteinsäureanhydrid- oder deren Säurenverhältnis zwischen 20 : 1 und 250 : 1 liegt.

14. Verfahren nach Anspruch 13, **dadurch gekennzeichnet, daß** das Verhältnis zwischen 40 : 1 und 100 : 1 liegt.

15. Verfahren nach einem der Ansprüche 1 bis 14, **dadurch gekennzeichnet, daß** die Hydrierung in einem Temperaturbereich von etwa 100 bis 400 °C, bevorzugt in einem Temperaturbereich von etwa 260 bis 320 °C durchgeführt wird.

16. Verfahren nach einem der Ansprüche 1 bis 15, **dadurch gekennzeichnet, daß** der überschüssige Wasserstoff rezirkuliert wird, nachdem die Reaktionsprodukte auskondensiert wurden.

17. Verfahren nach Anspruch 16, **dadurch gekennzeichnet, daß** die Kondensation bei einer Temperatur zwischen 0°C und 40°C erfolgt.

18. Verfahren nach einem der Ansprüche 1 bis 17, **dadurch gekennzeichnet, daß** man den Katalysator durch Reduktion im Reaktor vorbereitet.

19. Verfahren nach einem der Ansprüche 1 bis 18, **dadurch gekennzeichnet, daß** man den eingesetzten Katalysator ganz oder teilweise mit Inertmaterial verdünnt.

20. Verwendung des bei einem der Verfahren gemäß einem der Ansprüche 1 bis 19 anfallenden Reaktionsgemisches ohne Abtrennung des gebildeten Wassers und der anderen Nebenprodukte zur Herstellung von 2-Pyrrolidon mit Ammoniak und N-Alkylpyrrolidonen mit Alkylaminen insbesondere N-Methylpyrrolidon durch Umsetzung von Monomethylamin in der Flüssigphase unter Druck.

## Claims

1. Process for the preparation of gamma-butyrolactone by means of catalytic hydrogenation of maleic anhydride, succinic anhydride or the acids thereof in the vapour phase in the presence of catalysts based copper oxide and aluminium oxide in the reduced form, **characterised in that** to carry out the reaction use is made of a catalyst which is based on 50 to 95 wt.% of copper oxide, 3 to 30 wt.% of aluminium oxide, and 0 to 25 wt.% of a binder.

2. A process according to claim 1, **characterised in that** the catalyst is based on 80 to 90 wt.% of copper oxide, 5 to 15 wt.% of aluminium oxide, and for the missing remainder up to 100 wt.%, of a binder.

3. A process according to either of claims 1 to 2, **characterised in that** the binder used is graphite.

4. A process according to claim 2 or 3, **characterised in that** the catalyst is based on 83.5 to 85.5 wt.% of copper oxide, 9-11 wt.% of aluminium oxide, and 4.5 to 6.5 wt.% of graphite.

5. A process according to either of claims 1 to 2, **characterised in that** the binder used is silica gel.

6. A process according to any one of claims 1 to 5, **characterised in that** the catalyst has a homogeneous structure.

7. A process according to any one of claims 1 to 6, **characterised in that** the catalyst is applied on a carrier.

8. A process according to any one of claims 1 to 7, **characterised in that** the hydrogenation is carried out in the presence of pure hydrogen.

9. A process according to any one of claims 1 to 8, **characterised in that** the hydrogenation is carried out in the presence of an inert gas as diluent.

10. A process according to claim 9, **characterised in that** the diluent used is nitrogen.

11. A process according to any one of claims 1 to 10, **characterised in that** the pressure is between 0.1 and 10 bar.

12. A process according to claim 11, **characterised in that** the pressure is 4-7 bar.

13. A process according to any one of claims 1 to 12, **characterised in that** the molar ratio hydrogen : maleic anhydride, succinic anhydride or the acids thereof is between 20 : 1 and 250 : 1.

14. A process according to claim 13, **characterised in that** the ratio is between 40 : 1 and 100 : 1.

15. A process according to any one of claims 1 to 14, **characterised in that** the hydrogenation is carried out in a temperature range of about 100 to 400°C, preferably in a temperature range of about 260 to 320°C.

16. A process according to any one of claims 1 to 15, **characterised in that** the excess hydrogen is recycled, after the reaction products were condensed out.

17. A process according to claim 16, **characterised in that** the condensation takes place at a temperature between 0 and 40°C.

18. A process according to any one of claims 1 to 17, **characterised in that** the catalyst is pretreated by reduction in the reactor.

19. A process according to any one of claims 1 to 18, **characterised in that** the catalyst employed is diluted wholly or in part with inert material.

20. Use of the reaction mixture obtained by one of the processes according to any one of claims 1 to 19 without separation of the formed water and the other side products for the preparation of 2-pyrrolidone with ammonia and N-alkylpyrrolidones with alkylamines, in particular N-methylpyrrolidone, by conversion of monomethylamine in the liquid phase under pressure.

## Revendications

1. Procédé de préparation de gamma-butyrolactone par hydratation catalytique de l'anhydride d'acide maléique, de l'anhydride d'acide succinique ou leurs acides en phase vapeur en présence de catalyseurs à base d'oxyde de cuivre et d'oxyde d'aluminium sous forme réduite, **caractérisé en ce que** l'on utilise pour la réalisation de la réaction un catalyseur composé à base de 50 à 95 % en poids d'oxyde de cuivre, 3 à 30 % en poids d'oxyde d'aluminium et de 0 à 25 % en poids d'un liant.

2. Procédé selon la revendication 1, **caractérisé en ce que** le catalyseur est composé à base de 80 à 90 % en poids d'oxyde de cuivre, 5 à 15 % d'oxyde d'aluminium, et pour le reste d'un liant pour former 100 %.

3. Procédé selon l'une des revendications 1 à 2, **caractérisé en ce que** l'on utilise du graphite comme liant.

4. Procédé selon la revendication 2 ou 3, **caractérisé en ce que** le catalyseur est constitué à base de 83,5 à 85,5 % en poids d'oxyde de cuivre, 9 à 11 % en poids d'oxyde d'aluminium et 4,5 à 6,5 % en poids de graphite.

5. Procédé selon l'une des revendications 1 à 2, **caractérisé en ce que** l'on utilise du gel de silice comme liant.

6. Procédé selon l'une des revendications 1 à 5, **caractérisé en ce que** le catalyseur présente une structure uniforme.

7. Procédé selon l'une des revendications 1 à 6, **caractérisé en ce que** le catalyseur est déposé sur un support.

8. Procédé selon l'une des revendications 1 à 7, **caractérisé en ce que** l'on réalise l'hydratation en présence d'hydrogène pur.

9. Procédé selon l'une des revendications 1 à 8, **caractérisé en ce que** l'on réalise l'hydratation en présence d'un gaz inerte comme diluant.

10. Procédé selon la revendication 9, **caractérisé en ce que** l'on utilise de l'azote comme diluant.

11. Procédé selon l'une des revendications 1 à 10, **caractérisé en ce que** la pression est de 0,1 à 10 bars.

12. Procédé selon la revendication 11, **caractérisé en ce que** la pression est située entre 4 et 7 bars.

13. Procédé selon l'une des revendications 1 à 12, **caractérisé en ce que** le rapport molaire hydrogène : anhydride d'acide maléique, anhydride d'acide succinique et leurs acides se situe entre 20 : 1 et 250 : 1.

14. Procédé selon la revendication 13, **caractérisé en ce que** le rapport se situe entre 40 : 1 et 100 : 1.

15. Procédé selon l'une des revendications 1 à 14, **caractérisé en ce que** l'hydratatio s'effectue dans une plage de température allant de 100 à 400°C environ, de préférence dans une plage de température allant d'environ 260 à 320°C.

16. Procédé selon l'une des revendications 1 à 15, **caractérisé en ce que** l'on remet en circulation l'hydrogène en excédent, une fois les produits de réaction condensés.

17. Procédé selon la revendication 16, **caractérisé en ce que** la condensation s'effectue à une température située entre 0°C et 40°C.

18. Procédé selon l'une des revendications 1 à 17, **caractérisé en ce que** l'on prépare le catalyseur par réduction dans le réacteur.

19. Procédé selon l'une des revendications 1 à 18, **caractérisé en ce que** l'on dilue le catalyseur mis en oeuvre entièrement ou partiellement avec une substance inerte.

20. Utilisation du mélange réactionnel apparaissant dans l'un des procédés selon l'une des revendications 1 à 19 sans élimination de l'eau formée et des autres sous-produits pour la préparation de la 2-pyrrolidone avec de l'ammoniac et des N-alkylpyrrolidones avec des alkylamines, en particulier la N-méthylpyrrolidone, par conversion de la monométhylamine en phase liquide sous pression.
